# EUROPEAN PATENT SPECIFICATION

(11) **EP 0 548 265 B1**
(45) Date of publication and mention of the grant of the patent: **06.12.1995**
(21) Application number: 91918167.7
(22) Date of filing: 19.09.1991
(51) Int. Cl.: A61K 7/075

(54) **MILD SHAMPOO COMPOSITIONS**
MILDE SHAMPOOZUSAMMENSETZUNGEN
COMPOSITIONS DE SHAMPOOING DOUX

(30) Priority: 21.09.1990 US 586466; 05.04.1991 US 681015
(43) Date of publication of application: 30.06.1993
(73) Proprietor: THE PROCTER & GAMBLE COMPANY, Cincinnati, Ohio 45202 (US)
(72) Inventor: INMAN, Everett, Junior, Cincinnati, OH 45240 (US)
(74) Representative: Brooks, Maxim Courtney
(86) International application number: US9106786
(87) International publication number: WO9204882

(56) References cited:
- WO-A-91/11984
- US-A- 4 946 136
- US-A- 4 963 535

## Description

The present invention relates to mild shampoo compositions, which provide low irritation to skin and eyes while at the same time providing excellent cleaning and lathering, comparable to conventional shampoos. These shampoo compositions are particularly useful for shampooing the hair of children, or adults with sensitive scalps. Preferred compositions also provide excellent cosmetic in-use properties, including hair conditioning benefits.

Mild shampoo compositions which provide low irritation to the skin are highly desirable. Conventional shampoos contain high levels of harsh anionic surfactants. These materials can penetrate the skin and destroy its integrity. This results, at the very least, in rough skin, and can ultimately lead to red, irritated skin. Ideal shampoo compositions should provide sufficient lathering and cleaning benefits to cleanse the hair while at the same time causing little or no irritation to the skin. This is particularly essential for shampoo compositions used on babies, small children, or adults with dry or sensitive skin. Furthermore, children can have difficulty in getting shampoo in their eyes during the hair washing or rinsing process. Hence, mild shampoo compositions which also provide reduced eye sting are particularly desirable for use on children.

Mild shampoo compositions, in general, are well known in the art having been taught in, for example, EP-A-0250181, published December 23, 1987; US-A-4,578,216, Fujii et al., issued March 25, 1986; US-A-4,726,915, Verdicchio, issued February 23, 1988; GB-A-1,508,929, published April 26, 1978; EP-A 0160269, published November 6, 1985; US-A-4,435,300, Guth et al., issued March 6, 1984; US-A-4,426,310, Vernunica, issued January 17, 1984; US-A-3,950,417, Verdicchio et al., issued April 13, 1976; US-A-4,443,362, Guth et al., issued April 17, 1984; US-A-4,654,207, Preston, issued March 31, 1987; US-A-4,851,154, Grollier et al., issued July 25, 1989; US-A-4,292,212, Melby, issued September 29, 1981; and US-A-4,329,334, Su et al., issued May 11, 1982. These references teach the use of a wide variety of surfactant combinations to achieve mildness. These surfactants include mild anionic surfactants, such as ethoxylated alkyl sulfates, amphoteric surfactants, nonionic surfactants, and various combinations thereof.

A well-known product in the current market for mild shampoos is Johnson's Baby Shampoo (Johnson & Johnson). This product contains high levels of nonionic surfactant (PEG-80 Sorbitan Laurate), which is known to provide reduced eye sting. The formula is supplemented by some other mild surfactants to boost its cleaning performance (betaine, ethoxylated alkyl sulfate, an imidazoline-derived amphoteric, and an ethoxylated alkyl carboxylate).

The formulation of such mild shampoo compositions, however, generally is an exercise in efficacy and mildness trade-offs, with the resulting composition providing either good cleaning and lathering benefits, or mildness and low eye or skin irritation benefits. In fact, Johnson's Baby Shampoo, while being particularly mild to the skin and eyes, is not a very good cleaner and its lathering profile is not particularly robust.

Hence, it is an object of the present invention to provide a mild shampoo composition which provides good in-use characteristics such as lathering, both in terms of abundance of lather and lather stability, and cleaning, while at the same time providing skin mildness benefits, and preferably low eye sting benefits as well.

Also desirable in state of the art shampoo compositions are conditioning benefits inherent in the shampoo compositions. Such products are often termed two-in-one shampoos, meaning the compositions includes both cleaning and conditioning ingredients in the same product. Such compositions are difficult to formulate because the cleaning ingredients, in general, tend to be incompatible with the conditioning ingredients. One highly successful solution to this dilemma has been to use dispersed insoluble non-volatile silicone materials together with certain suspending agents for the silicones in shampoo compositions. Such technology is taught in US-A-4,704,272, Oh et al., issued November 3, 1987; US-A-4,741,855, Grote et al., issued May 3, 1988; and US-A-4,788,006, Bolich et al., issued June 5, 1984. One problem with the use of silicone materials to deliver hair conditioning from shampoo compositions is that such materials, in general, tend to suppress lathering of the composition. If silicones are used with robust high-lathering surfactants, such as most anionic surfactants, lathering may not be unduly affected. However, if such materials are used with milder, less robust lathering surfactants, lathering may well be suppressed to an unacceptable level from a consumer standpoint.

Hence, it is a further object of the present invention to provide a mild shampoo composition which provides excellent lathering and cleaning benefits while at the same time providing hair conditioning benefits through the use of dispersed insoluble non-volatile silicone conditioning agents.

These and other objects will become readily apparent from the detailed description which follows.

The present invention relates to mild shampoo compositions comprising:
(a) from 4% to 15% by weight of an anionic surfactant;
(b) from 0.5% to 6.0% by weight of an imidazolinium derivative of the formula: 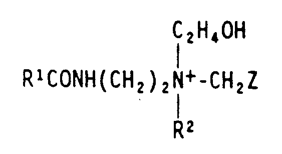 wherein R¹ is C₈ - C₂₂ alkyl or alkenyl, R² is hydrogen, CO₂M, CH₂CO₂M, or CH₂CH₂M, Z is CO₂M or CH₂CO₂M, and M is hydrogen, alkali metal, alkaline earth metal, ammonium or alkanol ammonium; (c) from 0.5% to 6.0% by weight of an amphoteric surfactant which is selected from the group consisting of aminoalkanoates of the formula:

   R-NH(CH₂)ₙCOOM

   iminodialkanoates of the formula:

   R-N[(CH₂)ₘCOOM]₂

   and mixtures thereof; wherein n and m are numbers from 1 to 4, R is C₈ - C₂₂ alkyl or alkenyl, and M is hydrogen, alkali metal, alkaline earth metal, ammonium or alkanol ammonium; and
(d) water;
wherein component a plus b plus c together comprise from 5% to 20% by weight of the composition and wherein the molar ratio of component a to components b plus c is from about 0.5:1 to about 2:1.

In another aspect of the present invention, the present compositions additionally comprise certain nonionic surfactants which help to mitigate the eye sting associated with the use of many anionic surfactants, such as ethoxylated lauryl sulfates. The preferred nonionic surfactants for mitigating eye sting while facilitating retention of the lather and viscosity attributes of the present compositions are polyethylene glycol glyceryl fatty esters, i.e., PEG glyceryl fatty esters. The PEG glyceryl fatty esters will generally have a PEG degree of polymerization of from about 5 to about 200, and the fatty ester will have an aliphatic hydrocarbyl radical of from about 8 to 20 carbons. The PEG glyceryl fatty esters will generally be of the formula: 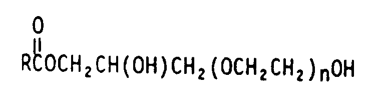
wherein n is from about 5 to about 200, preferably from about 20 to about 100, more preferably from about 30 to about 85, and RC(O)- is an ester wherein R comprises an aliphatic radical having from about 7 to 19 carbon atoms, preferably from about 9 to 17 carbon atoms, more preferably from about 11 to 17 carbon atoms, most preferably from about 11 to 14 carbon atoms. The combinations of n from about 20 to about 100, preferably from about 30 to about 85, to minimize eye sting, with C₁₂-C₁₈ (ie., n equals 11-17), preferably C₁₂-C₁₅ (ie., n equals 11-15) fatty esters, for minimized adverse effect on foaming, is highly preferred.

The essential, as well as optional, components of the present compositions are described below.

A critical component of the present compositions is an anionic surfactant material. This material is mainly responsible for the cleaning function of the present shampoo compositions.

Anionic surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols (C₈ - C₁₈ carbon atoms); sodium coconut fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms; sodium alkyl glyceryl ether sulfonates; the reaction product of fatty acids having from 10 to 22 carbon atoms esterified with isethionic acid and neutralized with sodium hydroxide; and others known in the art.

Such anionic surfactants tend to be quite harsh in terms of skin irritation and eye sting. The anionic surfactant component of the present invention is preferably chosen so as to be on the mild side. Examples of such milder anionic surfactants are the salts of sulfuric acid esters of the reaction product of 1 mole of a higher fatty alcohol and 1 to 12 moles of ethylene oxide, with sodium or ammonium being the preferred counterions. Particularly preferred are the alkyl sulfates containing about 3 moles of ethylene oxide, such as sodium laureth-3 sulfate or ammonium laureth-3 sulfate. Use of these anionic surfactants in the present compositions will provide optimized mildness benefits.

Depending on the degree of mildness desired in the present compositions from 0% to 100% of the anionic surfactant can comprise the ethoxylated alkyl sulfates. In other words, the more of this material that comprises the anionic surfactant, the milder the resulting composition will be.

Milder anionic surfactants may be used in the present compositions while still preserving the desired lather and cleaning profiles. These include the water soluble salts of condensation products of fatty acids with sarcosine, especially fatty acid sarcosinates derived from C₈ - C₂₂, preferably C₁₀ - C₁₈, fatty acids, most preferably sodium lauroyl sarcosinate (sold under the trade name Hamposyl-L-30 by Hampshire).

If the sarconsinates are present in the compositions of this invention, it is generally at a level of from about 1% to about 7%, preferably from about 2% to about 6%, by weight of the composition.

Of course these milder anionic surfactants tend to be more expensive, and for that reason are less desirable for use in hair shampoos. It may be desirable to use such materials in combination with the more traditional, albeit harsher, anionic surfactants, to enjoy at least some mildness benefits, while keeping the cost of the composition low.

Hence, the anionic surfactant component of the present invention is preferably selected from the group consisting of alkyl sulfates, alkyl sulfonates, ethoxylated alkyl sulfates, sarcosinates, and mixtures thereof.

The total anionic surfactant component is present in the mild shampoo compositions of the present invention at a level of from 4% to 15%, preferably from 5% to 11%, more preferably from 8% to 11% by weight.

Two additional critical components of the compositions of the present invention are selected from two particular classes of amphoteric surfactants.

The first class of amphoteric surfactants critical in the present invention is selected from certain imidazolinium derivatives. Such materials are depicted by Formula I: 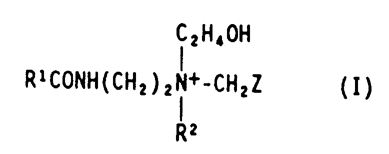
wherein R¹ is C₈ - C₂₂ alkyl or alkenyl, R² is hydrogen, CO₂M, CH₂CO₂M, or CH₂CH₂M, Z is CO₂M or CH₂CO₂M, and M is hydrogen, alkali metal, alkaline earth metal, ammonium or alkanol ammonium.

Suitable materials of this type are marketed under the tradename MIRANOL and are understood to comprise a complex mixture of species. The CTFA Cosmetic Dictionary, Third Edition, indicates Formula I as the formula for these materials. Traditionally, the Miranols have been described as having the following cyclic structure: 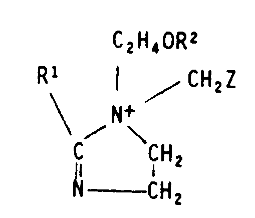
wherein R¹, R², and Z are defined as above, and M is hydrogen, alkali metal, alkaline earth metal, ammonium or alkanol ammonium. In practice, a complex mixture of species is likely to exist and hereinafter Formula I is intended to cover mixtures of species as defined above.

Materials preferred for use in the present compositions include cocoamphocarboxypropionate, cocoamphocarboxy propionic acid, and cocoamphocarboxyglycinate. Mixtures of these materials may also be used. The most preferred material of this type for use in the present invention is cocoamphocarboxyglycinate (also known as cocoamphodiacetate).

Specific commercial products providing the imidazolinium derivative component of the present compositions include those sold under the trade names of MIRANOL C2M CONC. N.P., MIRANOL C2M CONC. O.P., MIRANOL C2M SF, MIRANOL CM SPECIAL (Miranol, Inc.); ALKATERIC 2CIB (Alkaril Chemicals); AMPHOTERGE W-2 (Lonza, Inc.); MONATERIC CDX-38, MONATERIC CSH-32 (Mona Industries); REWOTERIC AM-2C (Rewo Chemical Group); and SCHERCOTIC MS-2 (Scher Chemicals).

The imidazolinium derivatives of the present invention exhibit low skin and eye irritation and also act to make other ingredients of the present compositions less irritating as well. Such materials demonstrate mildness, good lathering, foaming and cleansing, and excellent performance in the presence of oily soils, making them particularly useful in the shampoo compositions of the present invention.

The imidazolinium derivatives are present in the shampoo compositions of this invention at levels of from 0.5% to 6%, preferably from 2% to 4% by weight.

The second class of amphoteric surfactants critical in the present invention is selected from the group consisting of aminoalkanoates of Formula II:

R-NH(CH₂)ₙCOOM (II)

iminodialkanoates of Formula III:

R-N[(CH₂)ₘCOOM]₂ (III)

and mixtures thereof; wherein n and m are numbers from 1 to 4, R is C₈ - C₂₂ alkyl or alkenyl, and M is hydrogen, alkali metal, alkaline earth metal, ammonium or alkanolammonium.

Preferred examples of amphoteric surfactants falling within this first class include n-alkylamino-propionates and n-alkyliminodipropionates. Such materials are sold under the tradename DERIPHAT by Henkel and MIRATAINE by Miranol, Inc. Most preferred for use in the present compositions are N-lauryl-beta-amino propionic acid or salts thereof, and N-lauryl-beta-imino-dipropionic acid (DERIPHAT 160C) or salts thereof, and mixtures thereof.

The aminoalkanoates, iminodialkanoates, or mixtures thereof, are present in the compositions of this invention at a total level of from 0.5% to 6%, preferably from 1% to 4% by weight.

It is critical that both of these classes of amphoteric surfactants (imidazolinium derivatives plus aminoalkanoates and/or iminodialkanoates) are represented in the present compositions. Also critical are the following level and ratio limitations. The total level of anionic surfactant plus amphoteric surfactants in the present compositions is from 5% to 20%, preferably from 9% to 20%, more preferably from 9% to 18% by weight. Also, the molar ratio of anionic surfactant to total amphoteric surfactant in the present compositions is critically from about 0.5:1 to about 2:1. It is within these levels and ratios that the optimized mildness, lathering and cleaning benefits of the present compositions are provided. For compositions of the present composition formulated to deliver reduced eye sting benefits as well, the ratio of anionic surfactant to total amphoteric surfactant is preferably from about 0.8:1 to about 1.75:1, most preferably about 0.8:1 to about 1.5:1.

Water is the last essential component of the present invention and forms the remainder of the composition. It is generally present at a level of from 20% to 95%, preferably from 60% to 85% by weight of the composition.

The present compositions also optionally comprise certain nonionic surfactants which help to mitigate the eye sting associated with the use of many anionic surfactants, such as ethoxylated lauryl sulfates. The preferred nonionic surfactants for mitigating eye sting while facilitating retention of the lather and viscosity attributes of the present compositions are polyethylene glycol glyceryl fatty esters, i.e., PEG glyceryl fatty esters. The PEG glyceryl fatty ester will generally have a degree of polymerization of from about 5 to about 200, and the fatty ester will have an aliphatic hydrocarbyl radical of from about 8 to 20 carbons. The PEG glyceryl fatty esters will generally be of the formula: 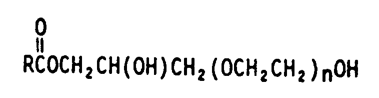
wherein n is from about 5 to about 200, preferably from about 20 to about 100, more preferably from about 30 to about 85, and RC(O)- is an ester wherein R comprises an aliphatic radical having from about 7 to 19 carbon atoms, preferably from about 9 to 17 carbon atoms, more preferably from about 11 to 17 carbon atoms, most preferably from about 11 to 14 carbon atoms. The combinations of n from about 20 to about 100, preferably from about 30 to about 85, to minimize eye sting, with C₁₂-C₁₈, preferably C₁₂-C₁₅ fatty esters, for minimized adverse effect on foaming, is highly preferred.

Suitable glyceryl fatty ester portions of these surfactants include glyceryl cocoate, glyceryl tallowate, glyceryl palmate, glyceryl stearate, glyceryl laurate, glyceryl oleate, glyceryl ricinoleate, and glyceryl fatty esters derived from triglycerides, such as palm oil, almond oil, and corn oil.

Preferred glyceryl esters include glyceryl tallowate and glyceryl cocoate.

Suitable surfactants of this class are commercially available from Sherex Chemical Co. (Dublin, Ohio, USA) under their Varonic® LI line of surfactants. These include, for example, Varonic LI 48 (polyethylene glycol (n=80) glyceryl tallowate, alternately referred to as PEG 80 glyceryl tallowate), Varonic LI 2 (PEG 28 glyceryl tallowate), Varonic LI 420 (PEG 200 glyceryl tallowate), and Varonic LI 63 and 67 (PEG 30 and PEG 80 glyceryl cocoates), and from Croda, Inc. (New York, New York, USA) under their Crovol® line of materials, such as Crovol A-40 (PEG 20 almond glyceride, Crovol A-70 (PEG 60 almond glyceride), Crovol M-40 (PEG 20 maize glyceride), Crovol M-70 (PEG 60 maize glyceride), Crovol PK-40 (PEG 12 palm kernel glyceride), and Crovol PK-70 (PEG-45 palm kernel glyceride). Especially preferred are monotallowate and cocoate fatty ester derivatives of polyethylene glycol, or mixtures thereof, particularly materials such as PEG 82 glyceryl monotallowate and PEG 30 glyceryl cocoate, and mixtures thereof.

The PEG glyceryl fatty ester nonionic surfactants are utilized at levels of from 2% to 20%, by weight, of the composition, preferably from 5% to 14%, more preferably from 7% to 11% by weight.

Other nonionic surfactants can also be used in the compositions hereof, though not necessarily for the purpose of reducing eye sting. These include, but are not limited to, alkylene oxide ethers of phenols, fatty alcohols, alkyl mercaptans, the alkylene oxide esters of fatty acid amides, the condensation products of ethylene oxide with partial fatty acid esters, and polysorbates, e.g., sucrose esters of fatty acids. Such materials are described in US-A-3,480,616, e.g., sucrose cocoate (a mixture of sucrose esters of a coconut acid, consisting primarily of monoesters, and sold under the tradenames GRILLOTEN LSE 87K from RITA, and CRODESTA SL-40 from Croda). Another class of nonionic materials that can be used is polyoxyethylene-polyoxypropylene copolymers, especially block copolymers, such as Pluronic F108 (CTFA name, Poloxamer 338 - a polyoxyethylene-polyoxypropylene block copolymer which conforms generally to the formula

HO(CH₂CH₂O)ₓ(CHCH₃CH₂O)_{y} (CH₂CH₂O)_{z}H,

wherein the average values of x, y, and z are respectively, 128, 54, and 128 - sold by BASF).

Still other nonionic surfactants may be included in the present compositions which are not necessarily included for the purpose of lowering eye sting, but which provide some other benefit in the compositions of the present invention. For example, nonionic surfactant materials may be included to further increase lathering without compromising the present skin mildness benefits. Suitable nonionic surfactants for this purpose include alkyl polysaccharides. Alkyl polysaccharide nonionic surfactants are disclosed in US-A-4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group. The polysaccharide can contain from about 1.0 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6-positions on the preceding saccharide units.

Optionally, and less desirably, there can be a polyalkyleneoxide chain joining the hydrophobic moiety and the polysaccharide moiety. The preferred alkyleneoxide is ethylene oxide. Typical hydrophobic groups include alkyl groups, either saturated or unsaturated, branched or unbranched containing from about 8 to about 18, preferably from about 10 to about 16, carbon atoms. Preferably, the alkyl group is a straight chain saturated alkyl group. The alkyl group can contain up to about 3 hydroxy groups and/or the polyalkyleneoxide chain can contain up to about 10, preferably less than 5, alkylene moieties. Suitable alkyl polysaccharides are octyl, nonyldecyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses. Suitable mixtures include coconut alkyl, di-, tri-, tetra-, and pentagluscosides and tallow alkyl, tetra-, penta-, and hexaglucosides.

The preferred alkyl polysaccharides are alkylpolyglycosides of the formula

R²O(CₙH₂ₙO)(glycosyl)ₓ

wherein R² is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 2 or 3, preferably 2; t is from 0 to about 10, preferably 0; and x is from 1.3 to about 10, preferably from 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

When included, nonionic surfactants, PEG glyceryl fatty ester generally are used at levels from 0.5% to 10%, more generally from 1% to 5% by weight, of the composition.

In addition to the required amphoteric surfactants described above, it may be desirable to include in the compositions hereof an optional amphoteric surfactant such as a sultaine, amidosultaine, or other surfactant. Sultaines and especially amidosultaines can advantageously be utilized as foam enhancing surfactants that are mild to the eye in partial replacement of anionic surfactants. Sultaines, including amidosultaines, include for example, cocodimethylpropylsultaine, stearyldimethylpropylsultaine, lauryl-bis-(2-hydroxyethyl) propylsultaine and the like; and the amidosultaines such as cocoamidodimethylpropylsultaine, stearylamidododimethylpropylsultaine, laurylamidobis-(2-hydroxyethyl) propylsultaine, and the like. Preferred are amidohydroxysultaines such as the C₁₂-C₁₈ hydrocarbyl amidopropyl hydroxysultaines, especially C₁₂-C₁₄ hydrocarbyl amido propyl hydroxysultaines, e.g., laurylamidopropyl hydroxysultaine and cocamidopropyl hydroxysultaine. Other sultaines are disclosed in US-A-3,950,417, issued April 13, 1976. Sultaines as described above are preferably used at levels of from 1% to 5%, more preferably from 2% to 3%, by weight, of the composition.

Preferred shampoo compositions of the present invention also comprise a dispersed, insoluble, non-volatile silicone conditioning agent. This material provides hair conditioning benefits such as ease of wet and dry hair combing and detangling, soft hair feel, and manageability to the user.

Silicone conditioning agents are known to cause lather suppression when used, in general, in shampoo compositions. With mild shampoo compositions silicones tend to negatively affect lather to even a greater degree. It has surprisingly been found that when non-volatile silicone conditioning agents are used in the present mild shampoo compositions comprising the unique above-identified surfactant systems, the lathering profile remains essentially unaffected.

The silicone hair conditioning agent used in the present invention is a siloxane or a siloxane-containing material and, when used, is present at a level of from 0.01% to 10% of the shampoo composition, preferably from 0.05% to 5%, more preferably from 0.05% to 3%, most preferably from 0.1% to 2.5% by weight.

Siloxanes (see, for example, US-A-3,208,911, Oppliger, issued September 28, 1965) and siloxane-containing polymers In general are known for use in hair conditioning compositions. US-A-4,601,902, Fridd et al., issued July 22, 1986, describes hair conditioning or shampoo/conditioner compositions which include a polydiorganosiloxane having quaternary ammonium substituted groups attached to the silicon, and a polydiorganosiloxane having silicon-bonded substituents which are amino-substituted hydrocarbon groups. US-A-4,654,161, Kollmeier et al., issued March 31, 1987, describes a group of organopolysiloxanes containing betaine substituents. When used in hair care compositions, these compounds are said to provide good conditioning, compatibility with anionic components, hair substantivity, and low skin irritation. US-A-4,563,347, Starch, issued January 7, 1986, relates to hair conditioning compositions which include siloxane components containing substituents to provide attachment to hair. JP-A-56-129,300, Lion Corporation, published October 9, 1981, relates to shampoo conditioner compositions which include an organopolysiloxaneoxyalkylene copolymer together with an acrylic resin. US-A-4,479,893, Hirota et al., issued October 30, 1984, describes shampoo conditioner compositions containing a phosphate ester surfactant and a silicon derivative (e.g., polyether- or alcohol-modified siloxanes). Polyether-modified polysiloxanes are also disclosed for use in shampoos in US-A-3,957,970, Korkis, issued May 18, 1976. US-A-4,185,087, Morlino, issued January 22, 1980, describes quaternary nitrogen derivatives of trialkylamino hydroxy organosilicon compounds which are said to have superior hair conditioning properties.

Non-volatile insoluble silicone fluids are useful as the silicone conditioning agent component in the shampoo compositions of the present invention. These materials provide maximum conditioning benefits from a shampoo composition because they are dispersed as a separate phase in the composition and, hence, deposit onto hair more readily from the composition. Examples of such materials include polydimethylsiloxane gums, aminosilicones and phenylsilicones. More specifically, materials such as polyalkyl or polyaryl siloxanes with the following structure: 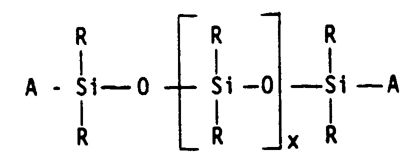
wherein R is alkyl or aryl, and x is an integer from about 7 to about 8,000, may be used. A represents groups which block the ends of the silicone chains, preferably -OH or C₁ - C₄ alkyl groups.

The alkyl or aryl groups substituted on the siloxane chain (R) or at the ends of the siloxane chains (A) may have any structure as long as the resulting silicones remain fluid at room temperature, are hydrophobic, are neither irritating, toxic nor otherwise harmful when applied to the hair, are compatible with the other components of the composition, are chemically stable under normal use and storage conditions, and are capable of being deposited on and of conditioning hair.

Suitable A groups include hydroxy, methyl, methoxy, ethoxy, propoxy, and aryloxy. The two R groups on the silicone atom may represent the same group or different groups. Preferably, the two R groups represent the same group. Suitable R groups include methyl, ethyl, propyl, phenyl, methylphenyl and phenylmethyl. The preferred silicones are polydimethylsiloxane, polydiethylsiloxane, and polymethylphenylsiloxane. Polydimethylsiloxane is especially preferred.

Suitable methods for preparing these silicone materials are disclosed in US-A-2,826,551 and US-A-3,964,500 and references cited therein. Silicones useful in the present invention are also commercially available. Suitable examples include Viscasil, a trademark of the General Electric Company, and silicones offered by Dow Corning Corporation and by SWS Silicones, a division of Stauffer Chemical Company.

The molecular weight of these silicone fluids can vary widely from liquid silicones having viscosities as low as about 10 mPa.s (centipoise), to silicone gums with viscosities of at least about 500,000 mPa.s (centipoise), at 25°C. Unless otherwise specifically stated, all viscosities shall be assumed to be determined at 25°C.

Other useful silicone conditioning materials include materials of the formula: 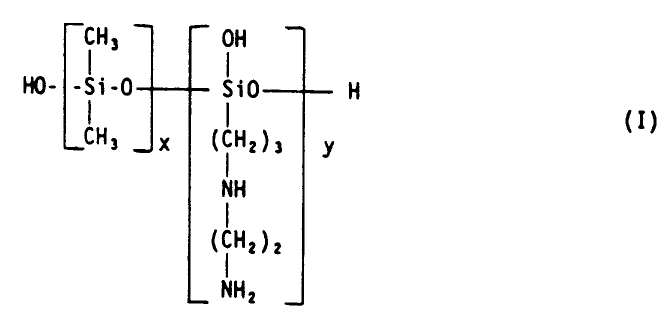
in which x and y are integers which depend on the molecular weight, the average molecular weight being approximately between 5,000 and 10,000. This polymer is also known as "amodimethicone".

Silicone cationic polymer conditioning agents which can also be used in the present compositions correspond to the formula:
(R₁)ₐG₃₋ₐ-Si-(-OSiG₂)ₙ-(OSiG_{b}(R₁)_{2-b})ₘ-O-SiG₃₋ₐ(R₁)ₐ in which G is chosen from the group consisting of hydrogen, phenyl, OH, C₁-C₈ alkyl, and preferably, methyl; a denotes 0 or an integer from 1 to 3, and preferably equals 0;
b denotes 0 or 1 and preferably equals 1; the sum n+m is a number from 1 to 2,000, and preferably from 50 to 150, n denoting a number from 0 to 1,999, and preferably from 49 to 149, and m denoting an integer from 1 to 2,000, and preferably from 1 to 10;
R₁ is a monovalent radical of formula C_{q}H_{2q}L in which q is an integer from 2 to 8 and L is chosen from the groups
-N(R₂)CH₂-CH₂-N(R₂)₂
-N(R₂)₂
-N⁺(R₂)₃A⁻
-N⁺(R₂)CH₂-CH₂-NR₂H₂A⁻
in which R₂ is chosen from the group consisting of hydrogen, phenyl, benzyl, a saturated hydrocarbon radical, preferably an alkyl radical containing from 1 to 20 carbon atoms, and A⁻ denotes a halide ion.

These compounds are described in greater detail in EP-A-95,238. An especially preferred polymer corresponding to this formula is the polymer known as "trimethylsilylamodimethicone" of the formula: 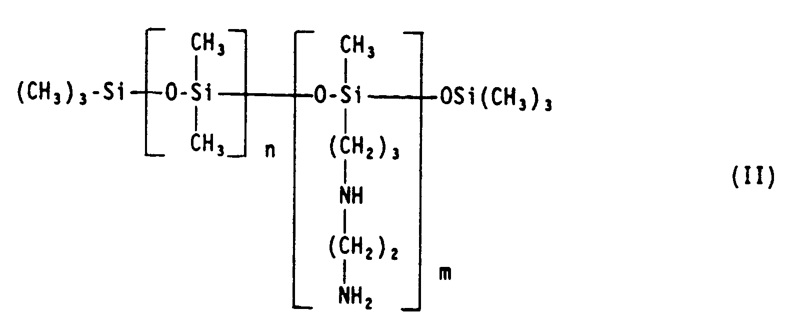
Other silicone cationic polymer conditioning agents which can be used in the present compositions correspond to the formula: 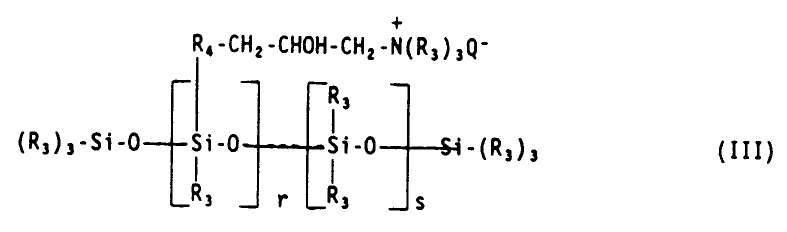
in which R₃ denotes a monovalent hydrocarbon radical having from 1 to 18 carbon atoms, and more especially an alkyl or alkenyl radical such as methyl;
R₄ denotes a hydrocarbon radical such as, preferably a C₁-C₁₈ alkylene radical or a C₁-C₁₈, and preferably C₁-C₈, alkyleneoxy radical;
Q⁻ is a halide ion, preferably chloride;
r denotes an average statistical value from 2 to 20, preferably from 2 to 8; and
s denotes an average statistical value from 20 to 200, preferably from 20 to 50.

These compounds are described in greater detail in US-A-4,185,017.

A polymer of this class which is especially preferred is that sold by UNION CARBIDE under the name "UCAR SILICONE ALE 56".

The efficacy of nonvolatile silicone hair conditioning agents can be enhanced through the use of silicone resin which is miscible with the silicone hair conditioning agent. Silicone resins are highly crosslinked polymeric siloxane systems. The crosslinking is introduced through the incorporation of trifunctional and tetrafunctional silanes with monofunctional or difunctional, or both, monomer units during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone resins will generally have at least about 1.1 oxygen atoms per silicon atom. Preferably, the ratio of oxygen: - silicon atoms is at least about 1.2:1.0. Typical silanes used in the manufacture of silicone resins are monomethyl-,dimethyl-, monophenyl-, diphenyl-, methylphenyl-, monovinyl-, and methyl-vinyl-chlorosilanes, and tetrachlorosilane. Preferred resins are the methyl substituted silicone resins, such as those offered by General Electric as GE SS4230 and SS4267. Commercially available silicone resins will generally be supplied in an unhardened form in a low viscosity volatile or nonvolatile silicone fluid. The silicone resins for use herein should be supplied and incorporated into the present compositions in such non-hardened form rather than as a hardened resin, as will be readily apparent to those skilled in the art.

The weight ratio of the nonvolatile silicone fluid conditioning component to the silicone resin component is preferably from about 4:1 to about 400:1. More preferably such ratio is from about 9:1 to about 200:1, most preferably from about 19:1 to about 100:1, particularly when the silicone fluid component is a polydimethylsiloxane fluid or a mixture of polydimethylsiloxane fluid and polydimethylsiloxane gum.

Other active hair care materials for use with the vehicle systems of the present invention are silicone polymer materials which provide both style retention and conditioning benefits to the hair. These include silicone polymers that are rigid silicone polymers. Such materials are described, for example, in US-A-4,902,499, Bolich et al., issued February 20, 1990.

Preferably the silicone conditioning agent comprises a mixture of a polydimethylsiloxane gum having a viscosity greater than about 1,000,000 mPa.s. (centipoise), and a dimethicone fluid having a viscosity of from about 10 mPa.s (centipoise) to about 100,000 mPa.s (centipoise), wherein the ratio of gum to fluid is from about 30:70 to about 70:30, preferably from about 40:60 to about 60:40.

Surprisingly, it has also been found that the compositions hereof having silicone conditioning agent, such as the preferred, insoluble silicones described above, have unexpectedly greater deposition levels on the hair when PEG glyceryl fatty ester nonionic surfactant is included in the formulation.

When the silicone conditioning agent is used in the present compositions and it is an insoluble silicone dispersed in the compositions, a suspending agent for the silicone will typically be required. Preferred suspending agents are long chain amine oxides and long chain acyl derivatives, such as those described US-A-4,741,855, Grote et al., issued May 3, 1988. Such suspending agents should be present in the composition in crystalline form. Especially preferred are mono- and di-ethylene glycol stearate compositions with less than about 7% monostearate. Alkanol amides of fatty acids, preferably having from about 16 to about 22, more preferably 16 to 18, carbon atoms (e.g., stearic mono- and di-ethanolamides, and stearic monoisopropanolamide, and stearic monoethanolamide stearate), and C₁₆-C₂₂ alkyl dimethyl amine oxides can also be used. Interestingly, although some of these materials, such as ethylene glycol distearate, may suppress lather in shampoo compositions, particularly in mild shampoo compositions, it has now been found that inclusion of this material in the present mild shampoo compositions surprisingly does not unduly suppress lather. This is yet another manifestation of the unique robustness of the lathering properties of the present shampoo compositions.

Another useful suspending agent for the silicones of the present compositions is xanthan gum as described in US-A-4,788,006, Bolich et al., issued June 5, 1984. Surprisingly, it has been found that xanthan gum can enhance lathering in the present compositions. The combination of long chain acyl derivatives and xanthan gum as a suspending system for silicone is described in US-A-4,704,272, Oh et al., issued November 3, 1987, and may also be used in the present compositions.

Another suspending agent that can be used, also an acyl derivative, includes N,N-di(hydrogenated) fatty hydrocarbyl (e.g., C₁₂-C₂₀ amido benzoic acids, and salts thereof, such as N,N-di(hydrogenated) C₁₆-C₁₈ amido benzoic acid, and salts thereof. Examples of materials of this type are N,N-di(hydrogenated) tallow amido benzoic acid, and the sodium and potassium salts thereof. Such materials can be obtained commercially from Stepan Company (Northfield, Illinois, USA). Other suspending agents known in the art can also be used.

Generally, from 0.1% to 5.0%, preferably from 0.1% to 3.0%, more preferably from 0.5% to 2.5% by weight, of the suspending agent is used to suspend the silicone in the present compositions.

The present compositions may also comprise other conventional shampoo components suitable for rendering such compositions more cosmetically or aesthetically acceptable or to provide them with additional usage benefits. These optional components generally are used individually in the compositions of the present invention at a level of from 0l01% to 10%, preferably from 0.05% to 5.0% by weight of the shampoo composition. Such conventional ingredients are well-known to those skilled in the art, e.g., pearlescent aids, such as TiO₂ coated mica; opacifiers; preservatives, such as benzyl alcohol, Glydant, Kathon, methyl paraben, propyl paraben, and imidazolidinyl urea; fatty alcohols, such as cetearyl alcohol, cetyl alcohol, and stearyl alcohol; sodium chloride; sodium sulfate; polyvinyl alcohol; ethyl alcohol;
pH adjusting aids, such as citric acid, sodium citrate, succinic acid, phosphoric acid, monosodium phosphate, disodium phosphate, sodium hydroxide, and sodium carbonate; coloring agents, such as any of the FD&C or D&C dyes; perfumes; sequestering agents, such as disodium ethylenediamine tetra-acetate; antidandruff aids; and pediculicides.

These optional components must be chosen so as not to interfere with the skin mildness benefits of the present invention. Preferably only ingredients which are not irritating to the eye are to be added. These materials also should be chosen so as not to interfere with the present cleaning and lathering benefits. Also, these additional components must be stable in the present shampoo compositions.

Though the silicone suspending agent component may act to thicken the present compositions to some degree, the present compositions may also optionally contain other thickeners and viscosity modifiers. Such thickeners must, of course, be chosen so as not to interfere with the benefits to be delivered by the present compositions. Examples of useful materials include water-soluble or colloidally water soluble polymers such as cellulose ethers (e.g., hydroxyethyl cellulose, methyl cellulose, hydroxypropylmethyl cellulose), polyvinylpyrrolidone, polyvinyl alcohol, guar gum, hydroxypropyl guar gum, dimethicone copolyols, starches, and starch derivatives. Various of these thickeners can be utilized as a primary suspension agent for silicone or be added at lower levels as a supplemental thickener.

Additionally, various materials can be added as foam enhancers. Foam enhancers are well known in the art. Preferred foam enhancers for use herein other than the optional amphoteric surfactant described above include materials such as xanthan gum, which can also serve as suspending agents for the silicone or other insoluble materials and cationic materials such as Polyquaternium-10 (an industry term designated by The Cosmetic, Toiletry and Fragrance Association (CTFA) for the polymeric quaternary ammonium salt of hydroxyethyl cellulose reacted with trimethyl ammonium substituted epoxide), commercially available from Union Carbide Corp. (Danbury, Connecticut, USA) under their UCARE POLYMER JR series of materials, e.g., UCARE POLYMER JR-30M, JR-125 and JR-400. Polyquaternium-10 is generally used at from 0.01% to 2%, preferably 0.05% to 1.5% by weight of the shampoo. Other preferred foam enhancing additives include alkylpolyglycosides as described above, and sultaines, especially amidohydroxypropyl sultaines, also described above.

Still other optional materials include antidandruff agents such as pyridinethione salts, specifically those in platelet form, as disclosed in US-A-4,379,753 and US-A-4,345,080. Included, for example, are heavy metal (e.g., zinc), magnesium, and aluminum salts of 1-hydroxy-2-pyridinethione. Other antidandruff agents include selenium compounds such as selenium disulfide. Antidandruff agents are normally used at levels of 0.1% to 4% of the composition, preferably 0.2% to 2% by weight.

Pediculicides can also be included in the compositions hereof to provide control of lice infestations. Suitable pediculicides are well known in the art and include, for example, pyrethrins such as those disclosed in US-A-4,668,666, Allan.

A significant benefit of the present invention is that mild shampoo compositions can be attained which retain excellent lathering in use. This is especially significant in the case of preferred embodiments also containing silicone conditioners, particularly nonvolatile silicone conditioners, which generally tend to reduce the lathering ability of shampoos. The present invention allows for shampoo compositions containing a sufficiently high level of anionic and amphoteric surfactants to compensate for the lather-reducing efforts of silicone conditioning agents, yet the surfactant systems of the present compositions remain mild. With the addition of PEG glyceryl fatty ester nonionic surfactants to mitigate eye sting, excellent mild shampoos containing silicone conditioning agent and having good foam and lather characteristics, as well as little or no eye sting, can be obtained. Surprisingly, these shampoos can be obtained at total anionic and amphoteric surfactant levels of in excess of 13%. The preferred levels of total anionic and amphoteric surfactant is from 15% to 20%, more preferably from 16% to 18% by weight. Such preferred compositions will more particularly, comprise from 5% to 11% by weight of the anionic surfactant, preferably from 8% to 11%, from 2% to 4% by weight of the amphoteric surfactant of Formula (I), from 1% to about 4% of the amphoteric surfactant of Formula (II) or (III), or a mixture thereof, from 0.1% to 5.0% by weight of a silicone conditioning agent, a suspending agent for the silicone conditioning agent if it is insoluble, and for compositions for which it is desired to mitigate eye sting, from 5% to 14%, preferably from 7% to 11% by weight, of the PEG glyceryl fatty ester nonionic surfactant.

The pH of the present compositions is from about 6.0 to about 7.5, preferably from about 6.5 to about 7.2. Buffering agents may be added to the compositions to achieve and maintain the desired pH of the composition. If the pH is outside of this range, eye sting may be increased. At the optimum pH, the amphoteric surfactants are at their ideal charge distribution to interact with the anionic surfactant and hence minimize the amount of irritating free surfactant in the product. The most pH for each combination of surfactants chosen may vary somewhat within this range, because of the varying isoelectric point for each of the amphoteric surfactants.

The shampoo compositions of the present invention may be manufactured using methods known in the art. Specific examples of methods for making are disclosed in the Examples to follow.

The shampoo compositions of the present invention are utilized conventionally, i.e., the hair is shampooed by applying a safe and effective amount of the mild shampoo composition to the scalp, massaging it in, and rinsing it out. The term a "safe and effective amount" as used herein, is an amount which is effective in cleaning the hair, while providing mildness benefits to the user, especially low skin and eye irritation.

The following examples illustrate the present invention.

All parts, percentages, and ratios herein are by weight unless otherwise specified. Some of the components come from suppliers as dilute solutions. The levels given reflect the active weight percent of such materials.

### Example I

The following is a mild shampoo composition representative of the present invention.

| Component | Weight % |
|---|---|
| Ammonium Laureth-3 Sulfate | 10.0 |
| Cocoamphodiacetate¹ | 3.5 |
| Sodium Lauriminodipropionate² | 3.5 |
| Alkylpolyglycoside³ | 3.0 |
| Ethylene Glycol Distearate | 3.0 |
| DMDM Hydantoin | 0.37 |
| Citric Acid | 0.50 |
| DRO Water⁴ | q.s. |
| | Total=100% |

| | |
|---|---|
| ¹ Available under the tradename MIRANOL C-2M from Miranol, Inc. | |
| ² Available under the tradename DERIPHAT 160C from Henkel, Inc. | |
| ³ A mixture of primarily C₁₄-C₁₆ alkyl polyglucosides having an average degree of polymerization of about 1.4; commercially available under the tradename APG 600 from Henkel, Inc. | |
| ⁴ Double reverse osmosis water | |

The composition is prepared as follows. The ammonium laureth-3 sulfate and water are placed in a tank and heated to 71°C. The mixture is agitated. The alkylpolyglycoside is added. The ethylene glycol distearate is then added and allowed to melt. The remaining ingredients (except the citric acid and preservatives) are then added. The mixture is passed through a high shear mixer and a heat exchanger where it is cooled to 38°C and collected in a finishing tank. Finally, the pH of the finished batch is adjusted to pH 6.5 - 7.2 with citric acid.

The composition provides excellent in-use and efficacy benefits such as cleaning and lathering, while at the same time providing excellent mildness benefits such as low skin irritation.

### Example II

The following is a mild shampoo composition representative of the present invention.

| Component | Weight % |
|---|---|
| Ammonium Laureth-3 Sulfate | 10.0 |
| Cocoamphodiacetate¹ | 3.5 |
| Sodium Lauriminodipropionate² | 3.5 |
| Alkylpolyglycoside³ | 3.0 |
| Ethylene Glycol Distearate | 3.0 |
| DMDM Hydantoin | 0.37 |
| Citric Acid | 0.50 |
| Cetyl Alcohol | 0.04 |
| Stearyl Alcohol | 0.01 |
| Dimethicone³ | 1.0 |
| DRO Water | q.s. |
| | Total=100% |

| | |
|---|---|
| ¹ Available under the tradename MIRANOL C-2M from Miranol, Inc. | |
| ² Available under the tradename DERIPHAT 160C from Henkel. | |
| ³ Available as APG 600 from Henkel, Inc. | |
| ⁴ A 40/60 blend of SE-76 gum from GE Silicones and a silicone fluid having a viscosity of about 350 centistokes. | |

The composition is prepared as follows. A premix is first prepared by adding a portion of the ammonium laureth-3 sulfate to a premix tank and heating to 71°C. The cetyl alcohol and stearyl alcohol are added and allowed to melt. The dimethicone is added and mixed until an emulsion is formed.

The remainder of the ammonium laureth-3 sulfate is placed in a separate tank. The mixture is agitated and heated to 71°C. The ethylene glycol distearate is added and allowed to melt. The remaining ingredients (except the citric acid) are then added. The main mix is passed through a high shear mixer and a heat exchanger where it is cooled to 38°C and collected in a finishing tank. The premix is also sheared, cooled, and collected in the same finishing tank, where the main mix and premix are mixed until homogeneous. Finally, the pH of the finished batch is adjusted to 6.5 - 7.2 with citric acid.

The composition provides excellent in-use and efficacy benefits such as cleaning and lathering, while at the same time providing excellent mildness benefits such as low skin irritation. The composition also provides hair conditioning in-use and end benefits.

### Example III

The following is a mild shampoo composition representative of the present invention.

| Component | Weight % |
|---|---|
| Sodium Laureth-3 Sulfate | 6.60 |
| Cocoamphodiacetate¹ | 2.70 |
| Disodium Lauriminodipropionate² | 2.70 |
| Polyethylene glycol (82) glyceryl monotallowate³ | 7.00 |
| Dimethicone⁴ | 1.53 |
| Cetyl Alcohol | 0.04 |
| Stearyl Alcohol | 0.01 |
| Ethylene Glycol Distearate | 2.00 |
| DMDM Hydantoin | 0.20 |
| Ethylene-diamine Tetra-acetic Acid (EDTA) | 0.10 |
| Citric Acid | 0.75 |
| DRO Water | q.s. |
| | Total=100% |

| | |
|---|---|
| ¹ Available under the tradename MIRAN0L C-2M from Miranol, Inc. | |
| ² Available under the tradename DERIPHAT 160C from Henkel. | |
| ³ Available under the tradename YAROHlC LI-48 by Sherex Chemical Company | |
| ⁴ A 40/60 blend of SE-76 silicone gum available from GE Silicones and a silicone fluid having a viscosity of about 350 centistokes. | |

The composition is prepared as follows. A premix is first prepared by adding a portion of the sodium laureth-3 sulfate to a premix tank and heating to 71°C. The cetyl alcohol and stearyl alcohol are added and allowed to melt. The dimethicone is added and mixed until an emulsion is formed.

The remainder of the laureth-3 sulfate, and a portion of the VARONIC LI-48 premixed in the DRO water are placed in a separate tank. The mixture is agitated and heated to 71°C. The ethylene glycol distearate is added and allowed to melt. The remaining ingredients (except the citric acid, preservative and remainder of the Varonic LI-48) are then added to provide the "main mix". The main mix is passed through a high shear mixer and a heat exchanger where it is cooled to 38°C and collected in a finishing tank. The sodium laureth-3-sulfate premix is also sheared, cooled, and collected in the same finishing tank, where the main mix and said premix are mixed until homogeneous. Finally, the pH of the finished batch is adjusted to 6.5 to 7.2 with citric acid.

The composition provides excellent in-use and efficacy benefits such as cleaning and lathering, while at the same time providing excellent mildness benefits such as low skin and eye irritation. The composition also provides hair conditioning in-use and end benefits.

### EXAMPLES IV-VIII

The following are mild shampoo compositions representative of the present invention

| Component (ppm or %, by weight, of composition) | IV | V | VI | VII | VIII |
|---|---|---|---|---|---|
| Sodium Laureth-3 Sulfate (%) | 10.00 | 10.00 | 8.60 | 10.00 | 10.00 |
| Cocoamphodiacetate (%)³ | 3.50 | 3.50 | 3.00 | 3.50 | 3.50 |
| Lauriminodipropionate (%)⁴ | 3.50 | 3.50 | 3.00 | 3.50 | 3.50 |
| Cocamidopropyl Hydroxysultaine (%) | | | 2.70 | | |
| PEG-82 Glyceryl Tallowate (%)⁵ | 6.00 | 6.00 | 6.00 | 6.00 | 6.00 |
| PEG-30 Glyceryl Cocoate (%)⁶ | 4.00 | 4.00 | 4.00 | 4.00 | 4.00 |
| Polyquaternium 10 (%)¹ | | | | 0.10 | 0.05 |
| Ethylene Glycol Distearate (%) | 2.00 | 1.00 | 2.00 | 2.00 | 2.00 |
| Xanthan Gum (%)⁷ | 0.10 | 0.10 | | | |
| Dimethicone (%)² | 0.40 | 0.40 | 0.30 | 0.40 | 0.40 |
| Perfume (%) | 0.40 | 0.40 | 0.40 | 0.40 | 0.40 |
| Ethylenediamine Tetra-acetic Acid (Na salt) (%) | 0.10 | 0.10 | 0.10 | 0.10 | 0.10 |
| DMDM Hydantoin (%) | 0.20 | 0.20 | 0.20 | 0.20 | 0.20 |
| Citric Acid (%) | 0.25 | 0.25 | 0.25 | 0.25 | 0.25 |
| Sodium Chloride (ppm) | 184 | 184 | 184 | 184 | 184 |
| SDA #40 Alcohol (ppm) | 150 | 150 | 150 | 150 | 150 |
| Color (ppm) | 8 | 8 | 8 | 8 | 8 |
| DRO Water | q. s. to 100% | | | | |

| | | | | | |
|---|---|---|---|---|---|
| ¹ UCARE Polymer JR-30M, commercially available from Union Carbide Corporation. | | | | | |
| ² A 40/60 blend of SE-76 silicone gum available from GE Silicones and a silicone fluid having a viscosity of about 350 centistokes. | | | | | |
| ³ Available under the tradename MIRANOL C-2M from Miranol, Inc. | | | | | |
| ⁴ Available under the tradename DERIPHAT 160C from Henkel, Inc. | | | | | |
| ⁵ Available under the tradename VARONIC LI-48 from Sherex Chemical Company. | | | | | |
| ⁶ Available under the tradename VARONIC LI-63 from Sherex Chemical Company. | | | | | |
| ⁷ Available under the tradename KETROL SF from Kelco Division of Merck and Company. | | | | | |

The compositions are prepared as follows. A silicone premix is first prepared by adding a portion of the sodium laureth-3 sulfate to the premix tank and heating to 71°C. A portion of the Varonic LI-48 and the sodium chloride are added and allowed to melt. The dimethicone is added and mixed until an emulsion is formed.

The remainder of the sodium laureth-3 sulfate, a portion of the Varonic LI-48, the cocoamphodiacetate, the lauriminopropionate, and the perfume are placed in a separate tank. The mixture (the "main mix") is agitated and heated to 71°C. The ethylene glycol distearate is then added and allowed to melt. The main mix is passed through a high shear mixer and a heat exchanger where it is cooled to 38°C and collected in a finishing tank. The premix is also sheared, cooled, and collected in the same finishing tank, where the main mix and the premix are mixed until homogeneous. Finally, the remainder of the ingredients are added and mixed into the shampoo composition. The final pH is adjusted by the citric and to within the range of 6.5 to 7.2.

## Claims

1. A mild shampoo composition characterized in that it comprises:
(a) from 4% to 15% by weight of an anionic surfactant;
(b) from 0.5% to 6.0% of an imidazolinium derivative of the formula: 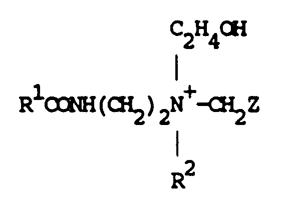 wherein R¹ is C₈ - C₂₂ alkyl or alkenyl, R² is hydrogen, CO₂M, CH₂CO₂M, or CH₂CH₂M, Z is CO₂M or CH₂CO₂M, and M is hydrogen, alkali metal, alkaline earth metal, ammonium or alkanol ammonium;
(c) from 0.5% to 6.0% of a material selected from the group consisting of aminoalkanoates of the formula:
R-NH(CH₂)ₙCOOM
iminodialkanoates of the formula:
R-N[(CH₂)ₘCOOM]₂
and mixtures thereof; wherein n and m are numbers from 1 to 4, R is C₈ - C₂₂ alkyl or alkenyl, and M is hydrogen, alkali metal, alkaline earth metal, ammonium or alkanol ammonium;
and
(d) water;
wherein component a plus b plus c together comprise from 5% to 20% by weight of the composition and wherein the molar ratio of component a to components b plus c is from 0.5:1 to 2:1.

2. The composition of Claim 1 wherein the anionic surfactant is present in the composition at from 5% to 11% by weight, and preferably comprises an ethoxylated alkyl sulfate, more preferably sodium laureth-3 sulfate.

3. The composition of Claim 1 or 2 wherein component b is selected from the group consisting of cocoamphocarboxypropionate. cocoamphocarboxy propionic acid, cocoamphocarboxyglycinate, and mixtures thereof, and preferably component b comprises cocoamphocarboxyglycinate.

4. The composition of Claim 1, 2, or 3 wherein component c is selected from the group consisting of n-alkylamino propionic acid and salts thereof, n-alkyliminodipropionic acid and salts thereof, and mixtures thereof, and preferably component c is selected from the group consisting of N-lauryl-beta-amino propionic acid and salts thereof, N-lauryl-beta-amino-dipropionic acid and salts thereof, and mixtures thereof, and most preferably c comprises N-lauryl-beta-imino-dipropionic acid, or salts thereof.

5. The composition of Claim 1, 2, 3, or 4 further characterized in that it additionally comprises from 0.01% to 10.0%, preferably from 0.05% to 5.0% by weight, of a dispersed insoluble non-volatile silicone conditioning agent, or mixtures thereof, wherein the silicone conditioning agent preferably comprises a combination of a polydimethyl siloxane gum having a viscosity, at 25°C, greater than 1,000,000 mPa.s (centipoise) and a dimethicone fluid having a viscosity, at 25°C, of from 10 mPa.s (centipoise) to 100,000 mPa.s (centipoise), the ratio of gum to fluid being from 30:70 to 70:30, preferably from 40:60 to 60:40.

6. The composition of Claim 5 wherein the level of silicone conditioning agent is from 0.05% to 5.0%, and a plus b plus c total at least 13%, by weight, of the composition.

7. The composition of Claim 1, 2, 3, 4, 5, or 6 having a pH of from 6.0 to 7.5.

8. A mild shampoo composition characterized in that it comprises:
(a) from 5% to 11% by weight of sodium laureth-3 sulfate;
(b) from 2% to 4% by weight of cocoamphocarboxy-glycinate;
(c) from 1% to 4% by weight of N-lauryl-beta-iminodipropionic acid, or salts thereof;
(d) from 0.05% to 5.0% by weight of a dispersed, insoluble, non-volatile silicone conditioning agent comprising a combination of a polydimethyl siloxane gum having a viscosity greater than 1,000,000 mPa.s (centipoise), at 25°C and a dimethicone gum having a viscosity of from 10 mPa.s (centipoise) to 100,000 mPa.s (centipoise), at 25°C, the ratio of gum to fluid being from 40:60 to 60:40;
(e) from 0.5% to 2.5% by weight of a suspending agent for the silicone conditioning agent selected from the group consisting of ethylene glycol distearate, xanthan gum, and mixtures thereof;
(f) water;
wherein component a plus b plus c together comprise from 13% to 20% by weight of the composition and wherein the molar ratio of component a to components b plus c is from 0.5:1 to 2:1, and the pH of the composition is from 6.0 to 7.5.

9. A mild shampoo composition characterized in that it comprises:
(a) from 4% to 15% by weight of an anionic surfactant, preferably from 5% to 11% by weight, of an anionic surfactant selected from the group consisting of alkyl sulfates, alkyl sulfonates, ethoxylated alkyl sulfates, sarcosinates, and mixtures thereof, and more preferably comprises sodium laureth-3 sulfate;
(b) from 0.5% to 6.0%, preferably from 2% to 4% by weight, of an imidazolinium derivative of the formula: 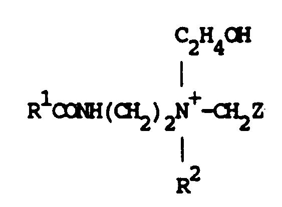 wherein R¹ is C₈ - C₂₂ alkyl or alkenyl, R² is hydrogen, CO₂M, CH₂CO₂M, or CH₂CH₂M, Z is CO₂M or CH₂CO₂M, and M is hydrogen, alkali metal, alkaline earth metal, ammonium or alkanol ammonium;
(c) from 0.5% to 6.0%, preferably from 1% to 4% by weight, of a material selected from the group consisting of aminoalkanoates of the formula:
R-NH(CH₂)ₙCOOM
iminodialkanoates of the formula:
R-N[(CH₂)ₘCOOM]₂
and mixtures thereof; wherein n and m are numbers from 1 to 4, R is C₈ - C₂₂ alkyl or alkenyl, and M is hydrogen, alkali metal, alkaline earth metal, ammonium or alkanol ammonium; and
(d) from 2% to 20% by weight of a polyethylene glycol glyceryl fatty ester nonionic surfactant; and
(e) water;
wherein component a plus b plus c together comprise from 5% to 20%, preferably at least 13%, more preferably from about 15% to about 20% by weight, of the composition and wherein the molar ratio of component a to component b plus c is from 0.5:1 to 2:1, preferably from 0.8:1 to 1.5:1.

10. The composition of Claim 9, wherein said polyethylene glycol glyceryl fatty ester has the formula: 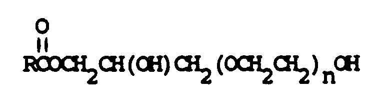 wherein n is from 20 to 100, preferably from 30 to 85, and R is an aliphatic alkyl or alkenyl radical having from 9 to 17 carbon atoms, preferably from 11 to 15 carbon atoms.

11. The composition of Claim 9 or 10 wherein component b is selected from the group consisting of cocoamphocarboxypropionate, cocoamphocarboxypropionic acid, cocoamphocarboxyglycinate, and mixtures thereof, preferably cocoamphocarboxyglycinate.

12. The composition of Claim 9, 10, or 11 wherein component c is selected from the group consisting of n-alkylamino propionic acid and salts thereof, n-alkyliminodipropionic acid and salts thereof, and mixtures thereof, preferably component c is selected from the group consisting of N-lauryl-beta-amino propionic acid and salts thereof, N-lauryl-beta-imino-dipropionic acid and salts thereof, and mixtures thereof, more preferably component c comprises N-lauryl-beta-imino-dipropionic acid, or salts thereof.

13. The composition of Claim 9, 10, 11, or 12, characterized in that it further comprises from 0.01% to 10.0%, preferably from 0.05% to 3.0% by weight, of a dispersed insoluble non-volatile silicone conditioning agent, or mixtures thereof, wherein the silicone conditioning agent comprises a combination of a polydimethyl siloxane gum having a viscosity greater than 1,000,000 mPa.s (centipoise), at 25°C, and a dimethicone fluid having a viscosity of from 10 mPa.s (centipoise) to 100,000 mPa.s (centipoise), at 25°C, the ratio of gum to fluid being from 30:70 to 70:30.

14. The composition of Claim 13 characterized in that it additionally comprises from 0.1% to 3% by weight of a suspending agent for the silicone conditioning agent, the suspending agent preferably being selected from the group consisting of ethylene glycol distearate, xanthan gum, and mixtures thereof.

15. The composition of Claim 9, 10, 11, 12, 13, or 14 wherein the pH is from 6.5 to 7.2.

16. The composition of Claim 1, 2, 3, 4, 5, 6, 7, 8, 9, 10, 11, 12, 13, 14, or 15 further comprising a foam enhancing material selected from the group consisting of alkylpolyglycoside nonionic surfactants, Polyquaternium-10, C₁₂-C₁₈ hydrocarbyl amidopropyl hydroxysultaine amphoteric surfactants, and xanthan gum, preferably said foam enhancing material is selected from the group consisting of alkylpolyglucoside nonionic surfactants, Polyquaternium-10, C₁₂-C₁₄ amidopropyl hydroxysultaines, and xanthan gum.

## Patentansprüche

1. Milde Shampoozusammensetzung, **dadurch gekennzeichnet**, daß sie umfaßt:
(a) 4 bis 15 Gew.-% eines anionischen Tensids;
(b) 0,5 bis 6,0 % eines Imidazoliniumderivats der Formel: 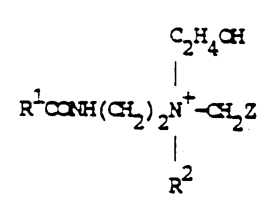 worin R¹ C₈-C₂₂-Alkyl oder -Alkenyl ist, R² Wasserstoff, CO₂M, CH₂CO₂M, oder CH₂CH₂M ist, Z CO₂M oder CH₂CO₂M ist und M Wasserstoff, Alkalimetall, Erdalkalimetall, Ammonium oder Alkanolammonium ist;
(c) 0,5 bis 6,0% eines Materials, gewählt aus der Aminoalkanoate der Formel
R-NH(CH₂)ₙCOOM
Iminodialkanoate der Formel:
R-N[(CH₂)ₘCOOM]₂
und Mischungen davon umfassenden Gruppe; worin n und m Zahlen von 1 bis 4 sind, R C₈-C₂₂-Alkyl oder -Alkenyl ist und M Wasserstoff, Alkalimetall, Erdalkalimetall, Ammonium oder Alkanolammonium ist; und
(d) Wasser;
wobei Komponente a + b + c zusammen 5 bis 20 Gew.-% der Zusammensetzung ausmachen und wobei das molare Verhältnis von Komponente a zu den Komponenten b + c 0,5:1 bis 2:1 beträgt.

2. Zusammensetzung nach Anspruch 1, wobei das anionische Tensid in der Zusammensetzung in einer Menge von 5 bis 11 Gew.-% vorliegt und vorzugsweise ein ethoxyliertes Alkylsulfat, weiter vorzugsweise Natriumlaureth-3-sulfat umfaßt.

3. Zusammensetzung nach Anspruch 1 oder 2, wobei die Komponente b aus der Cocoamphocarboxypropionat, Cocoamphocarboxypropionsäure, Cocoamphocarboxyglycinat und Mischungen davon umfassenden Gruppe gewählt ist, und wobei die Komponente b vorzugsweise Cocoamphocarboxyglycinat umfaßt.

4. Zusammensetzung nach Anspruch 1, 2 oder 3, wobei die Komponente c aus der n-Alkylaminopropionsäure und Salze davon, n-Alkyliminodipropionsäure und Salze davon und Mischungen davon umfassenden Gruppe gewählt ist, und wobei die Komponente c vorzugsweise aus der N-Lauryl-beta-aminopropionsäure und Salze davon, N-Lauryl-beta-iminodipropionsäure und Salze davon und Mischungen davon umfassenden Gruppe gewählt ist, und wobei am meisten bevorzugt die Komponente c N-Lauryl-beta-iminodipropionsäure oder Salze davon umfaßt.

5. Zusammensetzung nach Anspruch 1, 2, 3 oder 4, weiterhin **dadurch gekennzeichnet**, daß sie zusätzlich 0,01 bis 10.0 %, vorzugsweise 0,05 bis 5,0 Gew.-% eines disrpergierten, unlöslichen, nichtflüchtigen Silicon-Konditioniermittels oder Mischungen davon umfaßt, wobei das Silicon-Konditioniermittel vorzugsweise ein Kombination aus einem Polydimethylsiloxangummi mit einer Viskosität bei 25°C von mehr als 1 000 000 mPa·s (centipoise) und einem Dimethiconfluid mit einer Viskosität bei 25°C von 10 mPa·s (centipoise) bis 100 000 mPa·s (centipoise) umfaßt, wobei das Verhältnis von Gummi zu Fluid 30:70 bis 70:30, vorzugsweise 40:60 bis 60:40, beträgt.

6. Zusammensetzung nach Anspruch 5, wobei der Gehalt an Silicon-Konditioniermittel 0,05 bis 5,0 % beträgt und a + b + c insgesamt mindestens 13 Gew.-% der Zusammensetzung ausmachen.

7. Zusammensetzung nach Anspruch 1, 2, 3, 4, 5 oder 6 mit einem pH von 6,0 bis 7,5.

8. Milde Shampoozusammensetzung, **dadurch gekennzeichnet**, daß sie umfaßt:
(a) 5 bis 11 Gew.-% Natriumlaureth-3-sulfat;
(b) 2 bis 4 Gew-% Cocoamphocarboxyglycinat;
(c) 1 bis 4 Gew.-% N-Lauryl-beta-iminodipropionsäure oder Salze davon;
(d) 0,05 bis 5,0 Gew.-% eines dispergierten, unlöslichen, nichtflüchtigen Silicon-Konditioniermittels, umfassend eine Kombination aus einem Polydimethylsiloxangummi mit einer Viskosität von mehr als 1 000 000 mPa·s (centipoise) bei 25°C und einem Dimethicongummi mit einer Viskosität von 10 mPa·s (centipoise) bis 100 000 mPa·s (centipoise) bei 25°C, wobei das Verhältnis von Gummi zu Fluid 40:60 bis 60:40 beträgt; (e) 0,5 bis 2,5 Gew.-% eines Suspendiermittels für das Silicon-Konditioniermittel, gewählt aus der Ethylenglykoldistearat, Xanthangummi und Mischungen davon umfassenden Gruppe:
(f) Wasser;
wobei Komponente a + b + c zusammen 13 bis 20 Gew.-% der Zusammensetzung ausmachen und wobei das molare Verhältnis von Komponente a zu den Komponenten b + c 0,5:1 bis 2:1 beträgt, und wobei der pH der Zusammensetzung 6,0 bis 7,5 beträgt.

9. Milde Shampoozusammensetzung, **dadurch gekennzeichnet**, daß sie umfaßt:
(a) 4 bis 15 Gew.-% eines anionischen Tensids, vorzugsweise 5 bis 11 Gew.-% eines anionischen Tensids, gewählt aus der Alkylsulfate, Alkylsulfonate, ethoxylierte Alkylsulfate, Sarcosinate und Mischungen davon umfassenden Gruppe, wobei es weiter vorzugsweise Natriumlaureth-3-sulfat umfaßt;
(b) 0,5 bis 6,0 %, vorzugsweise 2 bis 4 Gew.-% eines Imidazoliniumderivats der Formel 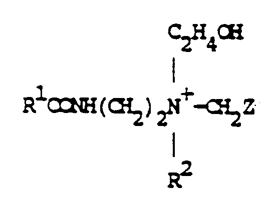 worin R¹ C₈-C₂₂-Alkyl oder -Alkenyl ist, R² Wasserstoff, CO₂M, CH₂CO₂M, oder CH₂CH₂M ist, Z CO₂M oder CH₂CO₂M ist und M Wasserstoff, Alkalimetall, Erdalkalimetall, Ammonium oder Alkanolammonium ist;
(c) 0,5 bis 6,0 %, vorzugsweise 1 bis 4 Gew.-% eines Materials, gewählt aus der Aminoalkanoate der Formel
R-NH(CH₂)ₙCOOM
Iminodialkanoate der Formel:
R-N[(CH₂)ₘCOOM]₂
und Mischungen davon umfassenden Gruppe; worin n und m Zahlen von 1 bis 4 sind, R C₈-C₂₂-Alkyl oder -Alkenyl ist und M Wasserstoff, Alkalimetall, Erdalkalimetall, Ammonium oder Alkanolammonium ist; und
(d) 2 bis 20 Gew.-% eines Polyethlyenglvkolglycerylfettsäureesters als nichtionisches Tensid; und
(e) Wasser;
wobei Komponente a + b + c zusammen 5 bis 20 Gew.-%, vorzugsweise mindestens 13 %, weiter vorzugsweise etwa 15 bis etwa 20 Gew.-% der Zusammensetzung ausmachen, und wobei das molare Verhältnis von Komponente a zu den Komponenten b + c 0,5:1 bis 2:1, vorzugsweiese 0,8:1 bis 1,5:1 beträgt.

10. Zusammensetzung nach Anspruch 9, wobei der Polyethylenglykolglycerylfettsäureester der Formel 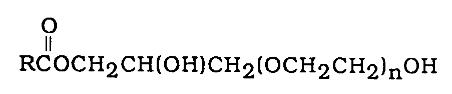 entspricht, worin n 20 bis 100, vorzugsweise 30 bis 85 ist, und R ein aliphatischer Alkyl- oder Alkenylrest mit 9 bis 17 Kohlenstoffatomen, vorzugsweise 11 bis 15 Kohlenstoffatomen ist.

11. Zusammensetzung nach Anspruch 9 oder 10, wobei Komponente b aus der Cocoamphocarboxypropionat, Cocoamphocarboxyprionsäure, Cocoamphocarboxyglycinat und Mischungen davon umfassenden Gruppe gewählt ist, vorzugsweise Cocoamphocarboxyglycinat ist.

12. Zusammensetzung nach Anspruch 9, 10 oder 11, wobei Komponente c aus der n-Alkylaminopropionsäure und Salze davon, n-Alkyliminodipropionsäure und Salze davon und Mischungen davon umfassenden Gruppe gewählt ist, und wobei die Komponente c vorzugsweise aus der N-Lauryl-beta-aminopropionsäure und Salze davon, N-Lauryl-beta-iminodipropionsäure und Salze davon und Mischungen davon umfassenden Gruppe gewählt ist, und wobei weiter bevorzugt die Komponente c N-Lauryl-beta-iminodipropionsäure oder Salze davon umfaßt.

13. Zusammensetzung nach Anspruch 9, 10, 11 oder 12, **dadurch gekennzeichnet**, daß sie weiterhin 0,01 bis 10,0%, vorzugsweise 0,05 bis 3,0 Gew.-% eines dispergierten, unlöslichen, nichtflüchtigen Silicon-Konditioniermittels oder Mischungen davon umfaßt, wobei das Silicon-Konditioniermittel vorzugsweise ein Kombination aus einem Polydimethylsiloxangummi mit einer Viskosität bei 25°C von mehr als 1 000 000 mPa·s (centipoise) und einem Dimethiconfluid mit einer Viskosität bei 25°C von 10 mPa·s (centipoise) bis 100 000 mPa·s (centipoise) umfaßt, wobei das Verhältnis von Gummi zu Fluid 30:70 bis 70:30 beträgt.

14. Zusammensetzung nach Anspruch 13, **dadurch gekennzeichnet**, daß sie weiterhin 0,1 bis 3 Gew.-% eines Suspendiermittels für das Silicon-Konditioniermittel umfaßt, wobei das Suspendiermittel vorzugsweise aus der Ethylenglykoldistearat, Xanthangummi und Mischungen davon umfassenden Gruppe gewählt ist.

15. Zusammensetzung nach Anspruch 9, 10, 11, 12, 13 oder 14, wobei der pH 6,5 bis 7,2 beträgt.

16. Zusammensetzung nach Anspruch 1, 2, 3, 4, 5, 6, 7, 8, 9, 10, 11, 12, 13, 14 oder 15, umfassend weiterhin ein schaumverstärkendes Material, gewählt aus der nichtionische Alkylpolyglycosid-Tenside, Polyquaternium-10, amphotere C₁₂-C₁₈-Hydrocarbylamidopropylhydroxysultain-Tenside und Xanthangummi umfassenden Gruppe, wobei das schaumverstärkende Material vorzugsweise aus der nichtionische Alkylpolyglycosid-Tenside, Polyquaternium-10, C₁₂-C₁₄-Amidopropylhydroxysultaine und Xanthangummi umfassenden Gruppe gewählt ist.

## Revendications

1. Composition de shampooing doux, caractérisée en ce qu'elle comprend:
(a) de 4% à 15% en poids d'un tensioactif anionique;
(b) de 0,5% à 6,0% d'un dérivé d'imidazolinium de formule: 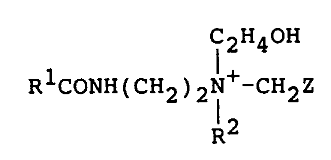 dans laquelle R¹ est un groupe alkyle ou alcényle en C₈-C₂₂, R² est un atome d'hydrogène ou un groupe CO₂M, CH₂CO₂M ou CH₂CH₂M, Z est CO₂M ou CH₂CO₂M, et M est un atome d'hydrogène, un métal alcalin, un métal alcalinoterreux, un ammonium ou un alcanolammonium;
(c) de 0,5% à 6,0% d'une substance choisie dans le groupe constitué par les aminoalcanoates de formule:
R-NH(CH₂)ₙCOOM
les iminodialcanoates de formule
R-N[(CH₂)ₘCOOM]₂
et leurs mélanges; dans lesquels n et m sont des nombres de 1 à 4, R est un groupe alkyle ou alcényle en C₈-C₂₂, et M est un atome d'hydrogène, un métal alcalin, un métal alcalino-terreux, un ammonium ou un alcanolammonium; et
(d) de l'eau;
dans laquelle les constituants a plus b plus c constituent ensemble de 5% à 20% en poids de la composition et dans laquelle le rapport molaire du constituant a aux constituants b plus c est de 0,5:1 à 2:1.

2. Composition selon la revendication 1, dans laquelle le tensioactif anionique est présent dans la composition à raison de 5% à 11% en poids et comprend de préférence un alkylsulfate éthoxylé, mieux encore du laureth-3 sulfate de sodium.

3. Composition selon la revendication 1 ou 2, dans laquelle le constituant b est choisi dans le groupe constitué par le coprah-amphocarboxypropionate, l'acide coprah-amphocarboxypropionique, le coprah-amphocarboxyglycinate et leurs mélanges, et de préférence le constituant b comprend du coprah-amphocarboxyglycinate.

4. Composition selon la revendication 1, 2 ou 3, dans laquelle le constituant c est choisi dans le groupe constitué par un acide n-alkylaminopropionique et ses sels, un acide n-alkyliminodipropionique et ses sels, et leurs mélanges, et de préférence le constituant c est choisi dans le groupe constitué par l'acide N-lauryl-β-aminopropionique et ses sels, l'acide N-lauryl-β-iminodipropionique et ses sels, et leurs mélanges, et mieux encore c comprend l'acide N-lauryl-β-iminodipropionique, ou ses sels.

5. Composition selon la revendication 1, 2, 3 ou 4, caractérisée en outre en ce qu'elle comprend aussi de 0,01% à 10,0%, de préférence de 0,05% à 5,0%, en poids, d'un agent de conditionnement silicone non volatil insoluble dispersé, ou de mélanges de ceux-ci, dans laquelle l'agent de conditionnement silicone comprend de préférence une combinaison d'une gomme de polydiméthylsiloxane ayant une viscosité, à 25°C, supérieure à 1 000 000 mPa.s (centipoises) et d'une huile de diméthicone ayant une viscosité, à 25°C, de 10 mPa.s (centipoises) à 100 000 mPa.s (centipoises), le rapport de la gomme à l'huile étant de 30:70 à 70:30, de préférence de 40:60 à 60:40.

6. Composition selon la revendication 5, dans laquelle la proportion d'agent de conditionnement silicone est de 0,05% à 5,0%, et le total de a plus b plus c constitue au moins 13%, en poids, de la composition.

7. Composition selon la revendication 1, 2, 3, 4, 5 ou 6, ayant un pH de 6,0 à 7,5.

8. Composition de shampooing doux, caractérisée en ce qu'elle comprend:
(a) de 5% à 11% en poids de laureth-3 sulfate de sodium;
(b) de 2% à 4% en poids de coprah-amphocarboxyglycinate;
(c) de 1% a 4% en poids d'acide N-lauryl-β-iminodipropionique, ou de ses sels;
(d) de 0,05% à 5,0% en poids d'un agent de conditionnement silicone non volatil insoluble, dispersé, comprenant une combinaison d'une gomme de polydiméthylsiloxane ayant une viscosité supérieure à 1 000 000 mPa.s (centipoises), à 25°C, et d'une huile de diméthicone ayant une viscosité de 10 mPa.s (centipoises) à 100 000 mPa.s (centipoises), à 25°C, le rapport de la gomme à l'huile étant de 40:60 à 60:40;
(e) de 0,5% à 2,5% en poids d'un agent de mise en suspension pour l'agent de conditionnement silicone, choisi dans le groupe constitué par le distéarate d'éthylèneglycol, la gomme xanthane, et des mélanges de ceux-ci;
(f) de l'eau;
dans laquelle les constituants a plus b plus c constituent ensemble de 13% à 20% en poids de la composition et dans laquelle le rapport molaire du constituant a aux constituants b plus c est de 0,5:1 à 2:1, et le pH de la composition est de 6,0 à 7,5.

9. Composition de shampooing doux, caractérisée en ce qu'elle comprend:
(a) de 4% à 15% en poids d'un tensioactif anionique, de préférence de 5% à 11% en poids d'un tensioactif anionique choisi dans le groupe constitué par les alkylsulfates, les alkylsulfonates, les alkylsulfates éthoxylés, les sarcosinates, et les mélanges de ceux-ci, et comprenant plus particulièrement le laureth-3 sulfate de sodium;
(b) de 0,5% à 6,0%, de préférence de 2% à 4%, en poids, d'un dérivé d'imidazolinium de formule: 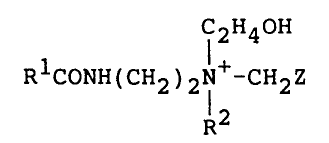 dans laquelle R¹ est un groupe alkyle ou alcényle en C₈-C₂₂, R² est un atome d'hydrogène ou un groupe CO₂M, CH₂CO₂M ou CH₂CH₂M, Z est CO₂M ou CH₂CO₂M, et M est un atome d'hydrogène, un métal alcalin, un metal alcalinoterreux, un ammonium ou un alcanolammonium;
(c) de 0,5% à 6,0%, de préférence de 1% à 4%, en poids, d'une substance choisie dans le groupe constitué par les aminoalcanoates de formule:
R-NH(CH₂)ₙCOOM
les iminodialcanoates de formule
R-N[(CH₂)ₘCOOM]₂
et leurs mélanges; dans lesquels n et m sont des nombres de 1 à 4, R est un groupe alkyle ou alcényle en C₈-C₂₂, et M est un atome d'hydrogène, un métal alcalin, un métal alcalino-terreux, un ammonium ou un alcanolammonium; et
(d) de 2% à 20% en poids d'un tensioactif non ionique de type ester gras glycérylique de polyéthylèneglycol; et
(e) de l'eau;
dans laquelle les constituants a plus b plus c constituent ensemble de 5% à 20%, de préférence au moins 13%, mieux encore d'environ 15% à environ 20%, en poids, de la composition et dans laquelle le rapport molaire du constituant a aux constituants b plus c est de 0,5:1 à 2:1, de préférence de 0,8:1 à 1,5:1.

10. Composition selon la revendication 9, dans laquelle ledit ester gras glycérylique de polyéthylèneglycol répond à la formule: 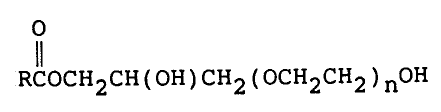 dans laquelle n a une valeur de 20 à 100, de préférence de 30 à 85, et R est un radical alkyle ou alcényle aliphatique comportant de 9 à 17 atomes de carbone, de préférence de 11 à 15 atomes de carbone.

11. Composition selon la revendication 9 ou 10, dans laquelle le constituant b est choisi dans le groupe constitué par le coprah-amphocarboxypropionate, l'acide coprah-amphocarboxypropionique, le coprah-amphocarboxyglycinate et leurs mélanges, de préférence le coprah-amphocarboxyglycinate.

12. Composition selon la revendication 9, 10 ou 11, dans laquelle le constituant c est choisi dans le groupe constitué par un acide n-alkylaminopropionique et ses sels, un acide n-alkyliminodipropionique et ses sels, et leurs mélanges, et de préférence le constituant c est choisi dans le groupe constitué par l'acide N-lauryl-β-aminopropionique et ses sels, l'acide N-lauryl-β-iminodipropionique et ses sels, et leurs mélanges, et mieux encore le constituant c comprend l'acide N-lauryl-β-iminodipropionique, ou ses sels.

13. Composition selon la revendication 9, 10, 11 ou 12, caractérisée en ce qu'elle comprend aussi de 0,01% à 10,0%, de préférence de 0,05% à 3,0%, en poids, d'un agent de conditionnement silicone non volatil insoluble dispersé, ou de mélanges de ceux-ci, dans laquelle l'agent de conditionnement silicone comprend une combinaison d'une gomme de polydiméthylsiloxane ayant une viscosité supérieure à 1 000 000 mPa.s (centipoises), à 25°C, et d'une huile de diméthicone ayant une viscosité de 10 mPa.s (centipoises) à 100 000 mPa.s (centipoises), à 25°C, le rapport de la gomme à l'huile étant de 30:70 à 70:30.

14. Composition selon la revendication 13, caractérisée en ce qu'elle comprend aussi de 0,1% à 3% en poids d'un agent de mise en suspension pour l'agent de conditionnement silicone, l'agent de mise en suspension étant de préférence choisi dans le groupe constitué par le distéarate d'éthylèneglycol, la gomme xanthane, et des mélanges de ceux-ci.

15. Composition selon la revendication 9, 10, 11, 12, 13 ou 14, dans laquelle le pH est de 6,5 à 7,2.

16. Composition selon la revendication 1, 2, 3, 4, 5, 6, 7, 8, 9, 10, 11, 12, 13, 14 ou 15, comprenant en outre une substance renforçatrice de moussage choisie dans le groupe constitué par les tensioactifs non ioniques de type alkylpolyglycoside, le Polyquaternium-10, les tensioactifs amphotères de type hydrocarbylamidopropylhydroxysultaïne en C₁₂-C₁₈ et la gomme xanthane, de préférence ladite substance renforçatrice de moussage est choisie dans le groupe constitué par les tensioactifs non ioniques de type alkylpolyglucoside, le Polyquaternium-10, les amidopropylhydroxysultaïnes en C₁₂-C₁₄ et la gomme xanthane.
